# EUROPEAN PATENT SPECIFICATION

(11) **EP 2 928 381 B1**
(45) Date of publication and mention of the grant of the patent: **26.04.2017**
(21) Application number: 13802576.2
(22) Date of filing: 04.12.2013
(51) Int. Cl.: A61B 10/00, A61B 10/02

(54) **DEVICE FOR OCULAR SAMPLING, PARTICULARLY FOR PERIPHERAL CORNEAL SAMPLING**
VORRICHTUNG ZUR OKULAREN ABTASTUNG, INSBESONDERE FÜR PERIPHERE HORNHAUTABTASTUNG
DISPOSITIF D'ÉCHANTILLONNAGE OCULAIRE, PARTICULIÈREMENT D'ÉCHANTILLONNAGE DE CORNÉE PÉRIPHÉRIQUE

(30) Priority: 05.12.2012 IT RM20120615
(43) Date of publication of application: 14.10.2015
(73) Proprietor: Holostem Terapie Avanzate S.r.l., 41125 Modena (MO) (IT)
(72) Inventor: DESOGUS, Michele, 41125 Modena (MO) (IT); PELLEGRINI, Graziella, 41051 Castelnuovo Rangone (MO) (IT)
(74) Representative: Capasso, Olga
(86) International application number: PCT/EP2013/075565
(87) International publication number: WO 2014/086878

(56) References cited:
- WO-A1-90/07902
- WO-A1-2010/100258
- JP-A- H0 670 933

## Description

The present invention relates to a device for ocular sampling, particularly suitable for peripheral corneal sampling.

The most common technique for corneal sampling requires the manual application of a buffer with filter in the central area of a patient's cornea.

However, this type of technique has several drawbacks and disadvantages. In fact, it only allows the detection of corneal dysfunctions at an advanced stage and moreover, it does not allow accurate diagnosis to be made in relation to the location of the pathological condition.

JP H06 70933 refers to a device for the extraction of epithelial cells of the cornea. Said device does not allow to achieve samples from different regions in one process step.

The object of the present invention is to provide a device for ocular sampling, particularly suitable for peripheral corneal sampling, which allows the drawbacks and disadvantages mentioned above with reference to the prior art to be overcome.

Another object of the invention is to provide a device of the above indicated type that is easily sterilizable.

A further object of the invention is to provide a device that is reliable to use, safe, multipurpose, modular, simple from the structural and economic point of view.

These objects are fully achieved by a device according to claim 1.

The device body further preferably comprises a lighting system whose light has the purpose of ensuring the correct positioning of the device during the diagnostic sampling.

According to a particular preferred embodiment of the present invention, the supporting pins of the device, as well as the sampling cylinders, are three. Such a device for ocular sampling, particularly for peripheral corneal sampling, advantageously allows very accurate and early diagnosis to be made.

Moreover, further advantages are offered by embodiments of the present invention claimed in the dependent claims.

Therefore, the present invention relates to a device according to claim 1.

Each of said at least two sampling cylinders is preferably coupled in a removable manner with one of the at least two supporting pins.

In a preferred embodiment, said device has a predetermined direction of preferential development (L) and said at least two sampling cylinders, when coupled with the at least two supporting pins, are inclined by an angle comprised between 10° and 25° with respect to said predetermined direction of preferential development (L).

Preferably, each membrane filter has pores ranging in diameter size between 0,4 and 3 micron. Said device has preferably a hole (5) opening into a top zone of said device concerned by the at least two supporting pins and comprises a light source communicating with said hole (5).

More preferably, the hole (5) opens into a substantially central region with respect to the arrangement of the at least two supporting pins.

Preferably, in the device according to the invention, the at least two supporting pins are three (4a, 4b, 4c) and the at least two sampling cylinders are three (7a, 7b, 7c).

More preferably, the supporting pins (4a, 4b, 4c), as well as the sampling cylinders (7a, 7b, 7c), are mutually arranged at angular distances of approximately 120° and/or the sampling cylinders (7a, 7b, 7c) are configured such that, when each is mounted on the respective supporting pins (4a, 4b, 4c), the centers of the membrane filters (9a, 9b, 9c) are positioned so as to describe substantially a circle with a diameter comprised between 10 and 20 mm.

Preferably, said circle has substantially a diameter of 15 mm.

Features and advantages of the present invention shall be better highlighted by the following description of a preferred but non-exclusive embodiment illustrated in the enclosed figures, having an illustrative and non-limiting purpose, wherein:

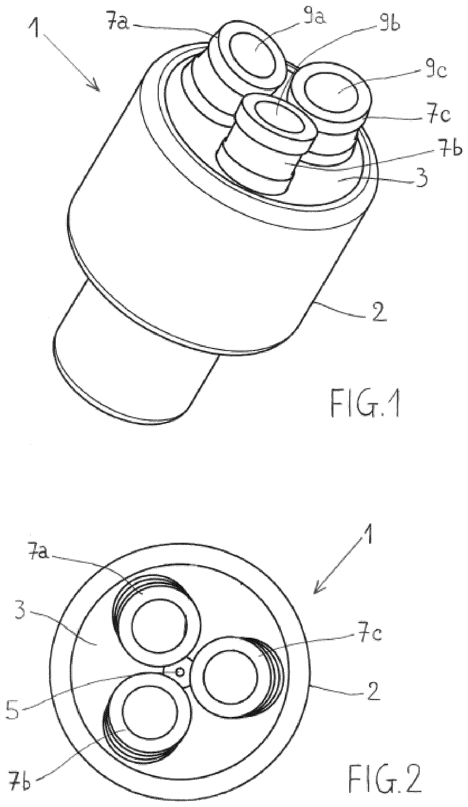

- figure 1 shows a perspective view of a device for ocular sampling according to the present invention, as a whole;
- figure 2 shows a top plan view of the device of figure 1;
- figure 3 shows the device according to the invention without the three removable sampling cylinders;
- figure 4 shows the device without the three removable sampling cylinders and related supporting pins;
- figure 5 shows a longitudinal section view of the device portion shown in figure 4;
- figure 6 shows one of the three supporting pins of the device according to the invention;
- figure 7 shows a longitudinal section view of the pin of figure 6;
- figure 8 shows a longitudinal section view of a sampling cylinder of the present device;
- figure 9 shows a top plan view of the sampling cylinder shown in figure 8.

With reference to the accompanying figures, reference numeral 1 indicates a device for ocular sampling, particularly for peripheral corneal sampling, according to the present invention.

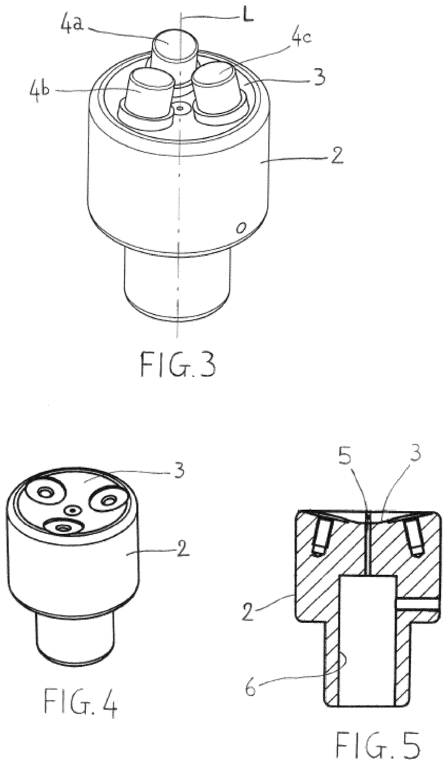

The device for ocular sampling 1 comprises a support 2, to allow the gripping thereof, with a predetermined preferential development direction L (figure 3) and a concave top surface 3 on which are assembled, peripherally and via threaded connection plugs, three supporting pins 4a, 4b, 4c preferably substantially cylindrical in shape.

The three supporting pins 4a, 4b, 4c are mutually arranged at angular distances of approximately 120°, converging towards said predetermined direction L and inclined, with respect to the latter, by an angle between 10° and 25°, preferably 12°.

The device for ocular sampling 1 also includes a light source, housed inside cavity 6, not shown in the accompanying figures for simplicity of representation, with an on and off device, for example a button, to facilitate the centering of the sampling on the ocular surface.

Said light source is in communication with a hole 5 leading in the top surface 3, made centrally on support 2 according to the predetermined direction L, and therefore in a central position with respect to the arrangement of the three supporting pins 4a, 4b, 4c.

Said hole 5, preferably but non limitedly, has a diameter of about 1.2 mm.

Moreover, a seat 6 is also provided in support 2 for housing one or more batteries for energy supply to the light source.

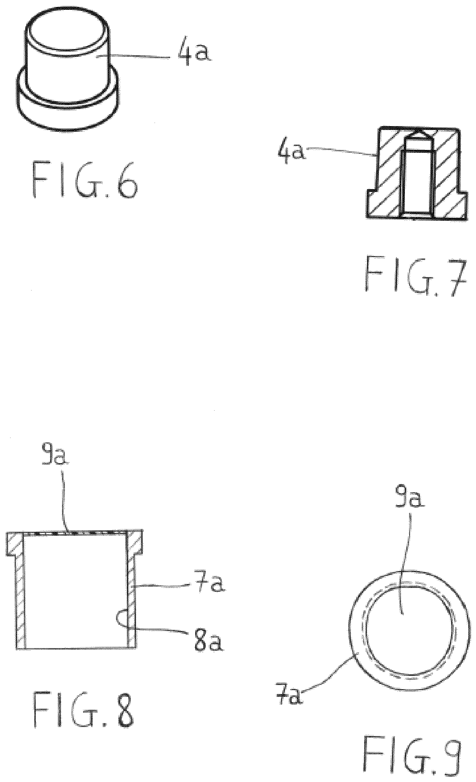

The device for ocular sampling 1 also comprises three sampling cylinders 7a, 7b, 7c, of sterile, or also disposable, type having a respective cavity (8a, in figure 8), each coupled, in a removable manner, to each of said supporting pins 4a, 4b, 4c, respectively, by simple shape coupling.

Each of the three sampling cylinders 7a, 7b, 7c includes, at the respective upper base, a membrane filter 9a, 9b, 9c, preferably made of polyethylene terephthalate (PET), communicating with the corresponding cavity (8 a, for cylinder 7a).

Preferably, the membrane filters 9a, 9b, 9c have pores ranging in diameter size between 0.4 and 3.0 micron.

The sampling cylinders 7a, 7b, 7c are identified and configured in such a way that, when mounted on the respective supporting pins 4a, 4b, 4c, the centers of the membrane filters 9a, 9b, 9c substantially describe a circle with a diameter between about 10 and 20 mm, preferably of about 15 mm.

The device for ocular sampling 1 described above is particularly suitable for use in impression limbal cytology.

In this case, the device for ocular sampling 1, once the membrane filters 9a, 9b, 9c have been inserted in the respective sampling cylinders 7a, 7b, 7c, is placed on a patient's eye with the indicated orientation (reference mark upwards) by matching the above filters with the three corresponding limbal areas, or sclero-corneal limbal areas, of the eye under examination, preferably after having applied one or more drops of topical anesthetic on the eye and having kept the relative eyelids open for at least ten seconds to allow the evaporation of the tear film and promote the adhesion of some layers of the epithelium to each membrane filter 9a, 9b, 9c. Before using the device for ocular sampling 1, the above-mentioned light bulb must be turned on in order to allow the centering between the main axis of the device and the eye axis passing by the pupil and therefore the precise application of the membrane filters 9a, 9b, 9c on the limbal areas of the eye.

Once the device for ocular sampling 1 has been brought in contact with the eye in the above described manner, it will be necessary to maintain the adhesion for at least 15 seconds and then firmly remove it from the corneal surface in order to allow the tearing of some epithelial layers and the relative sampling.

At this point, it will be possible to remove the sampling cylinders 7a, 7b, 7c from the respective supporting pins 4a, 4b, 4c and then to fix what collected on the membrane filters 9a, 9b, 9c by spraying a fixative for cytology on them, for example the Kito-Fix by the company Kaltek, composed of a mixture containing alcohol and polyethylene glycols, from a distance of about 15-20 cm.

Once the fixative has dried, it is advisable to verify the actual presence of one or more epithelial layers, which at this point should be clearly visible to the naked eye, on the membrane filters 9a, 9b, 9c.

Moreover, for an accurate diagnosis, it is also advisable to repeat the above described steps at least two more times, using a new set of membrane filters.

At this point, the membrane filters are ready to be sent to a laboratory for the analysis of epithelial layers adhering to them, where a preliminary staining of the membrane filters used for the sampling will be performed, using specific antibodies, and it will be subsequently verified whether the cornea or conjunctiva are present or not and, if so, in which areas of the filters. The alignment of the support with respect to the eye and the proper identification of supports and filters will allow the identification of the geographical area of origin of the analyzed tissue.

The device for ocular sampling 1 therefore allows to carry out simultaneously three sampling operations of epithelial layers in as many limbal areas of the eye.

In this way, it is therefore possible to sample the epithelium in multiple areas of the peripheral cornea rather than in the central cornea as provided by the traditional techniques, so as to be able to detect any corneal decompensation at an early stage, i.e. before the same has progressed to the central optical area of the eye.

Thanks to the use of the device object of the present invention, a surgeon can therefore decide whether to perform preventive therapeutic operations in order to block the advancement of the pathological condition.

According to further embodiments of the present invention, the device for ocular sampling proposed herein can advantageously comprise two or more sampling cylinders and as many supporting pins. Also in these cases, the device will still have to be applied to the eye to be examined by adhering the relative membrane filters to areas of the peripheral cornea.

A man skilled in the art may make several additional modifications and variants to the above described device for ocular sampling, particularly for peripheral corneal sampling, in order to meet further and contingent needs, all such modifications and variants falling within the scope of protection of the present invention, as defined by the annexed claims.

## Claims

1. A device for ocular sampling, particularly for peripheral corneal sampling, comprising:
- a support (2) from which at least two supporting pins extend, on a top thereof, and
- at least two sampling cylinders, each comprising, in correspondence with an end area, a membrane filter suitable, in use, to be brought in contact with an area of sclero-corneal limbus of an eye; and each of said at least two sampling cylinders being coupled, in a removable or not removable manner, with one of the at least two supporting pins.

2. The device according to claim 1, wherein said device has a predetermined direction of preferential development (L) and in which the at least two sampling cylinders, when coupled with the at least two supporting pins, are inclined by an angle comprised between 10° and 25° with respect to said predetermined direction of preferential development (L).

3. The device according to any of the previous claims, wherein each membrane filter has pores ranging in diameter size between 0,4 and 3 micron.

4. The device according to any of the previous claims, wherein said device has a hole (5) opening into a top zone of said device concerned by the at least two supporting pins and comprises a light source communicating with said hole (5).

5. The device according to claim 4, wherein the hole (5) opens into a substantially central region with respect to the arrangement of the at least two supporting pins.

6. The device according to any one of the previous claims, wherein the at least two supporting pins are three (4a, 4b, 4c) and the at least two sampling cylinders are three (7a, 7b, 7c).

7. The device according to claim 6, wherein the supporting pins (4a, 4b, 4c), as well as the sampling cylinders (7a, 7b, 7c), are mutually arranged at angular distances of approximately 120°.

8. The device according to claim 6 or 7, wherein the sampling cylinders (7a, 7b, 7c) are configured such that, when each is mounted on the respective supporting pins (4a, 4b, 4c), the centers of the membrane filters (9a, 9b, 9c) are positioned so as to describe substantially a circle with a diameter comprised between 10 and 20 mm.

9. The device according to claim 8, wherein the circle has substantially a diameter of 15 mm.

## Patentansprüche

1. Vorrichtung zur okularen Abtastung, insbesondere für die periphere Hornhautabtastung, umfassend:
- eine Halterung (2), von deren Oberseite sich zumindest zwei Haltestifte erstrecken, und
- zumindest zwei Abtastzylinder, die jeweils in Übereinstimmung mit einer Endfläche ein Membranfilter, umfassen, das bei Verwendung dazu ausgelegt ist, mit einem Bereich des sklerokornealen Limbus eines Auges in Kontakt gebracht zu werden; wobei jeder der zumindest zwei Abtastzylinder auf eine abnehmbare Weise oder eine nicht abnehmbare Wiese mit einem der zumindest zwei Haltestifte gekoppelt ist.

2. Vorrichtung nach Anspruch 1, wobei die Vorrichtung eine vorherbestimmte Richtung der bevorzugten Entwicklung (L) aufweist, und wobei die zumindest zwei Abtastzylinder dann, wenn sie mit den zumindest zwei Haltestiften gekoppelt sind, in Bezug auf die vorherbestimmte Richtung der bevorzugten Entwicklung (L) in einem Winkel geneigt sind, der zwischen 10° und 25° beträgt.

3. Vorrichtung nach einem der vorhergehenden Ansprüche, wobei jedes Membranfilter Poren mit einer Durchmessergröße in einem Bereich zwischen 0,4 und 3 Mikron aufweist.

4. Vorrichtung nach einem der vorhergehenden Ansprüche, wobei die Vorrichtung eine Öffnung (5) aufweist, die sich in eine von den zumindest zwei Haltestiften betroffene obere Zone der Vorrichtung öffnet, und eine Lichtquelle umfasst, die mit der Öffnung (5) in Verbindung steht.

5. Vorrichtung nach Anspruch 4, wobei sich die Öffnung (5) in Bezug auf die Anordnung der zumindest zwei Haltestifte in einen im Wesentlichen mittleren Bereich öffnet.

6. Vorrichtung nach einem der vorhergehenden Ansprüche, wobei die zumindest zwei Haltestifte drei (4a, 4b, 4c) sind, und die zumindest zwei Abtastzylinder (7a, 7b, 7c) drei sind.

7. Vorrichtung nach Anspruch 6, wobei die Stützstifte (4a, 4b, 4c) wie auch die Abtastzylinder (7a, 7b, 7c) untereinander in Winkelabständen von ungefähr 120° angeordnet sind.

8. Vorrichtung nach Anspruch 6 oder 7, wobei die Abtastzylinder (7a, 7b, 7c) derart ausgebildet sind, dass dann, wenn jeder davon an dem entsprechenden Haltestift (4a, 4b, 4c) angebracht ist, die Mitten der Membranfilter (9a, 9b, 9c) so positioniert sind, dass sie im Wesentlichen einen Kreis mit einem Durchmesser beschreiben, der zwischen 10 und 20 mm beträgt.

9. Vorrichtung nach Anspruch 8, wobei der Kreis im Wesentlichen einen Durchmesser von 15 mm aufweist.

## Revendications

1. Dispositif d'échantillonnage oculaire, en particulier pour l'échantillonnage de la cornée périphérique, comprenant :
- un support (2) à partir duquel s'étendent au moins deux broches de support, sur un dessus de celui-ci, et
- au moins deux cylindres d'échantillonnage, comprenant respectivement, en correspondance avec une zone terminale, un filtre à membrane approprié pour être mis en contact, pendant l'utilisation, avec une zone de limbe scléro-cornéen d'un oeil ; et chacun desdits au moins deux cylindres d'échantillonnage étant accouplé de façon amovible ou non-amovible à l'une des au moins deux broches de support.

2. Dispositif selon la revendication 1, dans lequel ledit dispositif présente une direction d'évolution préférentielle (L) prédéterminée, et dans lequel les au moins deux cylindres d'échantillonnage, lorsqu'ils sont accouplés aux au moins deux broches de support, sont inclinés d'un angle compris entre 10° et 25° par rapport à ladite direction d'évolution préférentielle (L).

3. Dispositif selon l'une quelconque des revendications précédentes, dans lequel chaque filtre à membrane présente des pores dont le diamètre varie entre 0,4 et 3 microns.

4. Dispositif selon l'une quelconque des revendications précédentes, dans lequel ledit dispositif présente un trou (5) débouchant sur une zone supérieure dudit dispositif concernée par les au moins deux broches de support, et comprend une source de lumière communiquant avec ledit trou (5).

5. Dispositif selon la revendication 4, dans lequel le trou (5) débouche sur une région substantiellement centrale par rapport à l'agencement des au moins deux broches de support.

6. Dispositif selon l'une quelconque des revendications précédentes, dans lequel les au moins deux broches de support sont trois (4a, 4b, 4c) et les au moins deux cylindres d'échantillonnage sont trois (7a, 7b, 7c).

7. Dispositif selon la revendication 6, dans lequel les broches de support (4a, 4b, 4c), ainsi que les cylindres d'échantillonnage (7a, 7b, 7c), sont mutuellement agencés à des distances angulaires d'environ 120°.

8. Dispositif selon la revendication 6 ou 7, dans lequel les cylindres d'échantillonnage (7a, 7b, 7c) sont configurés de telle façon, lorsque chacun est monté sur les broches de support (4a, 4b, 4c) respectives, que les centres des filtres à membrane (9a, 9b, 9c) sont positionnés de manière à décrire substantiellement un cercle avec un diamètre compris entre 10 et 20 mm.

9. Dispositif selon la revendication 8, dans lequel le cercle présente substantiellement un diamètre de 15 mm.
